# EUROPÄISCHE PATENTSCHRIFT

(11) **EP 0 659 444 B1**
(45) Veröffentlichungstag und Bekanntmachung des Hinweises auf die Patenterteilung: **26.05.1999**
(21) Anmeldenummer: 93120543.9
(22) Anmeldetag: 20.12.1993
(51) Int. Cl.: A61M 1/10

(54) **Vorrichtung zum Pumpen von Blut**
Apparatus for pumping blood
Appareil de pompage de sang

(43) Veröffentlichungstag der Anmeldung: 28.06.1995
(73) Patentinhaber: Stöckert Instrumente GmbH, D-80939 München (DE)
(72) Erfinder: Hahn, Andreas, Dipl.-Ing, D-82054 Sauerlach (DE)
(74) Vertreter: Ritter und Edler von Fischern, Bernhard, Dipl.-Ing.

(56) Entgegenhaltungen:
- EP-A- 0 156 211
- FR-A- 2 502 499
- US-A- 3 606 596
- US-A- 3 860 968
- US-A- 4 012 178
- US-A- 4 650 457
- IEEE TRANSACTIONS ON BIO-MEDICAL ENGINEERING, Bd.37, Nr.10, Oktober 1990, NEW YORK US Seiten 968 - 974 KITAMURA, GROSS 'ON-LINE PRESSURE ESTIMATION FOR A LEFT HEART ASSIST DEVICE'

## Beschreibung

Die vorliegende Erfindung betrifft eine Vorrichtung zum Pumpen von Blut oder anderen organisch-biologischen Flüssigkeiten.

Die Chirurgie des Herzens verlangt zumeist eine Eröffnung des Brustkorbes. Für kurz dauernde Eingriffe hat man die Möglichkeit, unter Hypothermie (Unterkühlung) die Blutzirkulation ganz aufzuheben. Größere Eingriffe verlangen die Aufrechterhaltung des Kreislaufs mit Hilfe einer Herz-Lungen-Maschine. Die verschiedenen Systeme haben unter anderem die Aufgabe, das Blut in Zirkulation zu halten, wozu Blutpumpen eingesetzt werden. Neben dieser Verwendung als Ersatz des natürlichen Kreislaufs bei stillgelegten Herzen eignen sich Blutpumpen auch zur Unterstützung eines geschwächten Herzens im Verlauf eines chirurgischen Eingriffs.

Vom physiologischen Standpunkt aus sollte die Umwälzung des Blutes in Anlehnung an den natürlichen Kreislauf pulsierend erfolgen, so daß auch beim künstlichen extrakorporalen Kreislauf eine weitgehend natürliche Versorgung des Organismus stattfindet. Aus diesem Grund sollten zur Aufrechterhaltung des künstlichen Kreislaufes über einen längeren Zeitraum pulsierende Blutpumpen eingesetzt werden.

Eine pulsierende Flüssigkeitspumpe für diesen Zweck ist bekannt aus CH 617 495 A5. Die Flüssigkeitspumpe besteht aus einem hermetisch verschlossenen Behälter, in den eine Druckblase aus flexiblem Material eingesetzt ist. Die Druckblase ist mit einem Ansauganschluß und einem Abgabeanschluß durch eine Wand des Behälters hindurch mit der Außenumgebung verbunden. In den beiden Anschlüssen befinden sich passive Rückschlagventile, die durch den in der Druckblase herrschenden Flüssigkeitsdruck gesteuert werden. Der mit einer Druckübertragungsflüssigkeit gefüllte Innenraum des Behälters ist mit einem pulsierenden Antrieb verbunden, wodurch die Druckblase im Rythmus des Antriebs komprimiert und expandiert wird. Die Druckblase kann nach Gebrauch weggeworfen und durch eine neue eresetzt werden, da sie gemäß einem Ausführungsbeispiel auf Stutzen an den beiden Anschlüssen aufgesteckt ist. Die beiden Anschlüsse und die darin angeordneten Rückschlagventile sind fest mit einer Wand des Behälters verbunden. In einem anderen Ausführungsbeispiel sind die Druckblase, die Anschlüsse und die passiven Rückschlagventile als Einheit ausgestaltet, wobei eine flache Wand der Druckblase direkt an die Druckübertragungsflüssigkeit angrenzt und so als Membran zwischen dem Innenraum der Druckblase und der Druckübertragungsflüssigkeit dient. Die Druckblase und die Druckübertragungskammer sind lösbar, jedoch aufwendig an einem Gehäuse befestigt. Auf die Druckübertragungsflüssigkeit wird mit Hilfe eines mechanisch aufwendigen Antriebs eingewirkt, um die pulsierende Flüssigkeitsströmung zu erzeugen.

Aus US-A-4 012 178 ist eine pulsierende Blutpumpe bekannt, bei der eine Druckblase in einem aufklappbaren Gehäuse angeordnet ist, das die Druckblase aufnimmt und in dessen Innenraum eine Druckübertragungsflüssigkeit eingebracht wird. Eine Kolbenpumpe expandiert und komprimiert über die Druckübertragungsflüssigkeit die Druckblase, die zwischen zwei nach außen führenden Anschlußstutzen eingespannt ist. In den Anschlußstutzen, die von außen den Anschluß von Zuleitungen gestatten, sind passive Kugel-Rückschlagventile ausgebildet, deren Aufbau so gestaltet ist, daß eine weitgehend laminare Strömung erzielt wird. Die beiden Rückschlagventile öffnen und schließen sich druckgesteuert entsprechend dem Druck, der von der Kolbenpumpe über die Druckübertragungsflüssigkeit auf das in der Druckblase befindliche Blut aufgebracht wird. Die Druckblase und die beiden Anschlußstutzen können aus dem Druckbehälter zusammenhängend entfernt werden, so daß nach Gebrauch der mit dem Blut in Berührung kommende Teil der Pumpe ausgewechselt werden kann.

Eine weitere pulsierende Blutpumpe ist aus DE-C-34 28 828 bekannt. Mit Hilfe einer Sekundärflüssigkeit wird der von einer intermittierend arbeitenden mechanischen Pumpe erzeugte Druck an einen von einem Dauermembran umschlossenen Raum weitergegeben. In diesem befindet sich das Blut, das direkt mengenproportional zur Kolbenbewegung der Pumpe aus dem Innenraum der Membran verdrängt wird. Neben einem berührungslosen Pumpvorgang ergibt sich eine regelbare Volumenströmung, da stets eine der Arbeitsflüssigkeit entsprechende Menge Blutes bewegt wird. Die sich einstellende Volumenströmung wird über einfaches Zählen der Arbeitsgänge der mechanischen Pumpe überwacht; der Druck des geförderten Blutes kann ohne Anordnung eines Drucksensors im Blutkreislauf bestimmt werden, da es genügt, den Druck der Arbeitsflüssigkeit zu bestimmen.

Die bekannten pulsierenden Blutpumpen erlauben es, einen künstlichen Blutkreislauf aufzubauen, bei dem aufgrund des pulsierenden Blutstroms bereits eine an den natürlichen Kreislauf angelehnte Versorgung des Organismus stattfindet.

Auch aus US-A-4 650 457 ist eine pulsierende Blutpumpe bekannt, mit der ein extrakorporaler Kreislauf aufgebaut werden kann, der bei stillgelegten Herzen den natürlichen Kreislauf des Patienten ersetzt. Im einzelnen weist die bekannte Blutpumpe ein flexibles Behältnis mit einem Einlaßanschluß und einem Auslaßanschluß auf, an die jeweils Schlauchleitungen des extrakorporalen Kreislaufs angeschlossen werden können. Das flexible Behältnis ist in einem druckdicht verschlossenen Gehäuse angeordnet, an das wiederum eine Pumpe angeschlossen ist, mit dem ein Druckmittel gefördert werden kann, das den Innendruck des Gehäuses erhöht bzw. absenkt. An den Anschlüssen bzw. Schlauchleitungen der Blutpumpe sind Klemmventile vorgesehen, die die Öffnungsquerschnitte der Anschlüsse bzw. der Schlauchleitungen bestimmen. Die Druckmittelpumpe und die Klemmventile sind an eine Steuereinheit angeschlossen, die die Pumpe und die Klemmventile entsprechend einem vorgegebenen Programm betreibt. Jedoch sind die Ventile bei dieser bekannten Blutpumpe lediglich dafür vorgesehen, die Schlauchleitungen abzuklemmen, um einen Rückfluß des Blutes in die Pumpe zu verhindern. Aus diesem Grund werden als Alternative für die aktiven Klemmventile in US-A-4 650 457 Rückschlagventile angegeben.

Eine weitergehende Annäherung an einen natürlichen Blutkreislauf ist bei den bekannten Systemen nahezu unmöglich, da eine Beeinflussung der Volumenströmung allein über den Druckantrieb oder Absperrventiele erfolgen kann. Probleme in verstärktem Maße entstehen beim Einsatz der bekannten Blutpumpen zur Unterstützung des nur geschwächten natürlichen Kreislaufs eines Organismus, denn die Anpassung muß dabei in Abhängigkeit vom zu unterstützenden Kreislauf erfolgen; die nur beschränkten Möglichkeiten fallen hier besonders ins Gewicht.

Davon ausgehend liegt der Erfindung die Aufgabe zugrunde, eine Vorrichtung zum Pumpen von Blut zu schaffen, bei der eine weitergehende Annäherung an einen natürlichen Blutkreislauf, insbesondere bei einem den natürlichen Kreislauf nur unterstützenden Einsatz möglich ist.

Gelöst wird diese Aufgabe durch eine Vorrichtung mit den Merkmalen des Patentanspruch 1. Vorteilhafte Ausgestaltungen ergeben sich aus den Unteransprüchen.

Erfindungsgemäß besitzt die Vorrichtung zum Pumpen von Blut neben einem pulsierenden Blutpumpenelement je ein aktives Klemmventil auf der Einlaß- und der Auslaßseite des Pumpenelements und zumindest einen Sensorabschnitt auf der Auslaßseite, in dem berührungslos arbeitende Druck- und Durchflußmengensensoren vorgesehen sind. Eine Steuereinheit registriert über die Sensorsignale Druck und Durchflußmenge und steuert die Klemmventile und eine Pumpe, die den Druck in einem Gehäuse des Blutpumpenelements erhöht bzw. erniedrigt. Ein im Gehäuse vorgesehenes flexibles Behältnis, vorzugsweise eine Druckblase wird durch die Druckänderungen komprimiert oder expandiert und fördert auf diese Weise pulsierend Blut. Schlauchanschlüsse, die an der Druckblase befestigt sind, führen aus dem druckdichten Gehäuse nach außen und zu dem Sensorabschnitt, sowie den Klemmventilen und gestatten den Anschluß des übrigen Kreislaufs.

Im folgenden wird die vorliegende Erfindung anhand eines Ausführungsbeispieles unter Bezugnahme auf die Figuren genauer beschrieben, in denen zeigt:
- Fig.1: den Aufbau der erfindungsgemäßen Vorrichtung;
- Fig.2: eine besondere Ausgestaltung des Gehäuses der Vorrichtung gemäß Fig. 1; und
- Fig. 3: ein Diagramm eines beispielhaften Druckverlaufs des geförderten Blutes bei einer erfindungsgemäßen Vorrichtung.

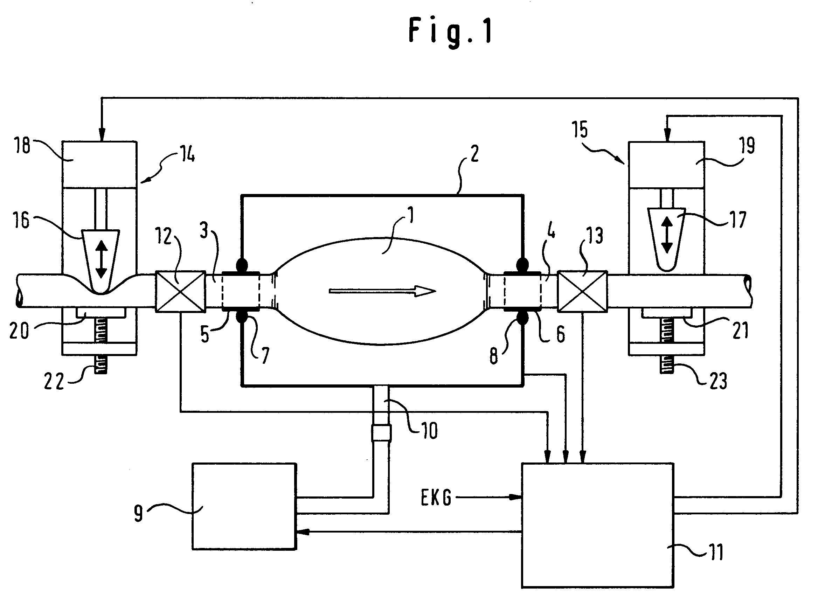

Bei dem in Fig. 1 gezeigten Ausführungsbeispiel der Erfindung ist eine elastische Druckblase 1 im Inneren eines hermetisch verschlossenen Gehäuses 2 angeordnet. Durch Öffnungen in gegenüberliegenden Wänden des Gehäuses 2 sind schlauchartige Anschlüsse 3 und 4 nach außen geführt. Die Anschlüsse 3 und 4 sind fest mit der Druckblase 1 verbunden, vorzugsweise einstückig mit der Druckblase 1 ausgebildet und im Bereich der Öffnungen des Gehäuses 2 außen mit Hülsen 5 und 6 ausgestattet, an denen Dichtelemente 7 und 8 des Gehäuses anliegen. Die Hülsen 5 und 6 besitzen eine Steifigkeit, die es in ausreichendem Maße gestattet, daß die Dichtelemente 7 und 8 so an den Hülsen anliegen, daß das Innere des Gehäuses 2 im Bereich der Öffnungen druckdicht abgeschlossen wird. Die Hülsen können aus Hartplastik hergestellt und auf die Anschlußschläuche aufgesteckt und verklebt oder in Form von integral ausgebildeten Schlauchabschnitten mit entsprechender Steifigkeit realisiert sein.

Eine Pumpe 9 ist an einem Druckmittelanschluß 10 des Gehäuses angeschlossen. Die Pumpe 9 dient zur Erzeugung eines Über- bzw. Unterdrucks im Inneren des Gehäuses 2 mit Hilfe eines gasförmigen oder flüssigen Druckübertragungsmittels. Dadurch kann die Druckblase 1 komprimiert oder expaniert und eine entsprechende Kraft auf das sich im Inneren der Druckblase befindliche Blut ausgeübt werden. Bei der erfindungsgemäßen Vorrichtung ist ein gasförmiges Druckübertragungsmittel eingesetzt, da durch die im folgenden noch genauer beschriebenen Steuerungs- und Regelmöglichkeiten keine Beziehung zwischen bewegtem Druckübertragungsmittel und gefördertem Blut bestehen muß, wie sie bei den bekannten Systemen erforderlich war. Die Verwendung eines gasförmigen Druckübertragungsmittels führt vielmehr zu einem wichtigen Vorteil der Erfindung. Eine Undichtigkeit der Druckblase 1 oder der Anschlußschläuche 3 und 4 läßt sich mit Hilfe eines Blasendetektors sicher erkennen, falls das Druckübertragungsmittel in den Blutkreislauf gelangt. Ein flüssiges Druckübertragungsmittel kann über einen nicht unerheblichen Zeitraum unbemerkt das Blut verunreinigen, bevor eine Leckage endeckt wird.

Die Pumpe 9 wird angesteuert von einer Steuereinheit 11, die im übrigen auch die Überwachung und Steuerung der gesamten Vorrichtung durchführt. Die Steuereinheit 11 ist dazu vorzugweise programmgesteuert, so daß abgespeicherte Steuerungsabläufe wiederholt abgerufen und ergänzt oder verändert werden können. Neue Steuerungsabläufe können hinzugefügt werden.

Bei dem gezeigten Ausführungsbeispiel sind an beiden Anschlußschläuchen 3 und 4 Sensorabschnitte 12 und 13 mit Sensoren zur Erfassung des Druckes und der Strömungsmenge des Blutkreislaufes vorgesehen. Jedoch kann die erfindungsgemäße Vorrichtung allein mit dem Sensorabschnitt 13 auf der Auslaßseite ausgeführt werden. Die Sensorabschnitte 12 und 13 können darüber hinaus einen Blasendetektor enthalten, mit dessen Hilfe, insbesondere auf der Auslaßseite, Gasblasen in dem geförderten Blut festgestellt werden können. Die in den Sensorabschnitten 12 und 13 vorgesehenen Sensoren oder Detektoren sind mit der Steuereinheit 11 verbunden.

Sowohl auf der Einlaßseite als auch auf der Auslaßseite ist bei der erfindungsgemäßen Vorrichtung ein aktives Klemmventil 14 bzw. 15 vorgesehen. Die Ansteuerung des Verschlußelements 16 bzw. 17 der Ventile erfolgt über einen elektrischen, elektromagnetischen, pneumatischen, hydraulischen oder anderen geeigneten Antrieb 18 bzw. 19, der das Verschlußelement geeignet, vorzugsweise linear zwischen zwei Endstellungen bewegt und der von der Steuereinheit 11 angesteuert wird. Die Verschlußelemente 16 und 17 wirken unmittelbar von außen auf die Anschlußschläuche 3 und 4 bzw. auf die daran angeschlossenen Schlauchstücke ein. Den beweglichen Verschlußelementen 16 und 17 sind Gegenlager 20 und 21 zugeordnet, deren Lage beispielsweise mit Hilfe einer Spindelschraube 22 bzw. 23 genau verstellbar ist, so daß sich die im Inneren des Schlauches einstellende Oklusion einstellen läßt. Blutschädigende Einflüsse können dadurch sicher vermieden werden.

Die Reihenfolge der Sensorabschnitte 12 und 13 und der aktiven Klemmventile 14 und 15 auf beiden Seiten des Pumpelements ist beliebig.

Anstelle einer elastischen Druckblase kann auch ein flexibler, z.B. faltbarer Behälter im Gehäuse vorgesehen werden, der mit den beiden Anschlußschläuchen verbunden ist.

Die erfindungsgemäße Vorrichtung bietet die Möglichkeit, das Schlagvolumen, die Schlagfrequenz sowie systolische und diastolische Werte zu erzeugen, die den physiologischen Werten des Herzens entsprechen. Die Steuereinheit 11 mißt dazu zumindest auf der Auslaßseite über den Sensorabschnitt 13, vorzugsweise aber auch auf der Einlaßseite mit Hilfe des Sensorabschnittes 12 den Druck und die Durchflußmenge. Sowohl die Ansteuerung der Pumpe 9 als auch der beiden Klemmventile 14 und 15 erfolgt derart, daß das den natürlichen Blutkreislauf kennzeichnende Druckprofil nachgefahren wird. Vorzugsweise erhält die Steuereinheit 11 ein Auslösesignal von einem (in Fig. 1 nicht dargestellten) EKG-Gerät.

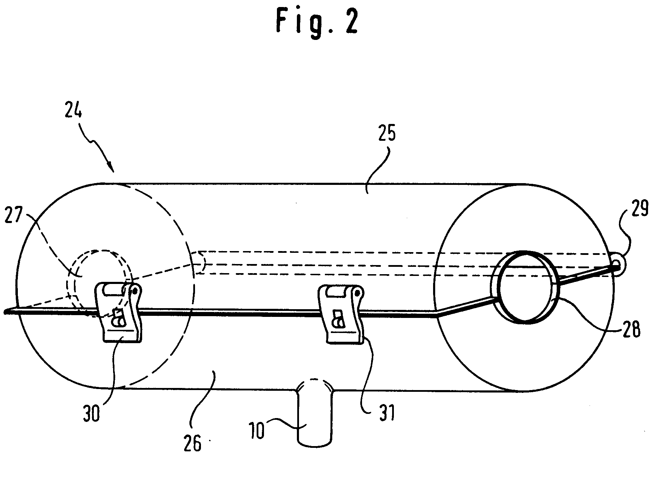

Wie in Fig 2 dargestellt, ist das Gehäuse 2 als durchsichtige, aufklappbare Kammer 24 realisierbar, die sich druckdicht verschließen läßt und, wie in Fig. 1 bereits gezeigt, die auch die beiden Hülsen 5 und 6 druckdicht umschließt. Sowohl die Kammer gemäß Fig. 2 als auch das in Fig. 1 gezeigte Gehäuse 2 eignen sich für einen pneumatischen oder hydraulischen Antrieb. Die Kammer 24 ist zweiteilig und besteht aus einem Deckelteil 25 und einem Bodenteil 26, die gemeinsam eine im wesentlichen zylindrische Grundform festlegen. An den Stirnenden sind die Öffnungen 27 und 28 für die nach außen führenden Schlauchanschlüsse vorgesehen. Sowohl am Rand der Öffnungen 27 und 28 als auch an den übrigen Rändern besitzen die beiden Teile der Kammer 24 Dichtelemente. Die beiden Teile sind über ein Scharnier 29 miteinander klappbar verbunden. Geeignete Verschlüsse 30 und 31 erlauben ein schnelles und problemloses Öffnen bzw. Verschließen der Druckkammer. Geeignet sind zu diesem Zweck beispielsweise Kipphebelverschlüsse. An der Bodenhälfte 26 der Kammer 24 ist der Anschluß 10 für die Pumpe vorgesehen. Darüber hinaus können Befestigungseinrichtungen an dieser Hälfte der Kammer angebracht werden, die es gestatten, die Kammer an einem Stativ anzubringen.

Das Material der Kammer ist vorzugsweise durchsichtig, so daß nicht nur der Pumpvorgang beobachtet, sondern auch mögliche Leckagen visuell erkannt werden können. An den Stirnflächen können die Sensorabschnitte und/oder die aktiven Klemmventile unmittelbar befestigt sein.

Neben der zuvor beschriebenen Ausgestaltung als aufklappbare Kammer kann ein zweiteiliges Gehäuse verwendet werden, daß nach Einsetzen der Druckblase fest verschlossen, beispielsweise verklebt wird. Das Gehäuse bildet dann mit dem flexiblen Behältnis in seinem Inneren eine Einheit, wodurch der Aufwand reduziert wird, der hinsichtlich der druckdichten Ausgestaltung eines zu öffnenden Gehäuses mit entnehmbarer Druckblase erforderlich ist.

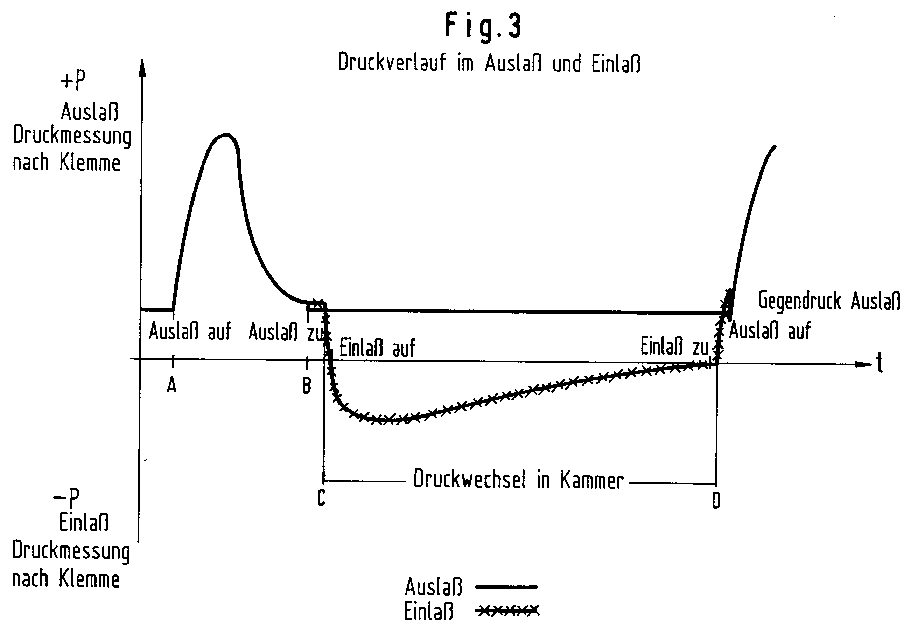

Fig. 3 zeigt den Druckverlauf in der Einlaß- und Auslaßschlauchleitung 3 bzw. 4. Der Sensorabschnitt 12 war bei dieser Messung in Stömungsrichtung hinter dem aktiven Einlaßklemmventil 14, der Sensorabschnitt 13 stromabwärts vom aktiven Auslaßklemmventil 15 angeordnet. Zum Zeitpunkt A wird von der Steuereinheit 11 das Klemmventil 15 geöffnet und die Pumpe 9 druckerhöhend angesteuert, was zunächst einen Druckanstieg zur Folge hat. Der Druck geht dann zurück, da das Blut aus der Druckblase 1 gegen den durch den Kreislauf des Patienten hervorgerufenen Gegendruck gefördert wird. Zum Zeitpunkt B wird das Auslaßklemmventil 15 durch die Steuereinheit 11 geschlossen. Der Druck hinter dem Klemmventil 15 bleibt daraufhin konstant. Zum Zeitpunkt C wird das Einlaßventil geöffnet und die Pumpe 9 druckabsenkend angesteuert, woraufhin zunächst der Druck in der Einlaßschlauchleitung 3 abfällt. D.h., zum Zeitpunkt C findet auch der Druckwechsel im Inneren des Gehäuses 2 statt. Bis zum Zeitpunkt D wird durch Erzeugung eines Unterdrucks im Gehäuse 2 Blut durch die Einlaßschlauchleitung 3 in die Druckblase 1 gefördert. Im Zeitpunkt D wird das Einlaßklemmventil 14 geschlossen und ein erneuter Ausstoßvorgang durch Öffnen des Auslaßklemmventils 15 und einen erneuten Druckwechsel der Pumpe 9 eingeleitet.

Alle Parameter des Blutkreislaufes lassen sich in weiten Grenzen festlegen. Durch entsprechende Ansteuerung der Pumpe 9 und der beiden aktiven Klemmventile 14 und 15 kann die Pumprate (Frequenz), der Druckverlauf sowie das Fördervolumen eingestellt werden. Die Steuereinheit 11 steuert dazu die Schließ- und Öffnungszeiten und -geschwindigkeiten der Klemmventile 14 und 15 sowie die Druckbeaufschlagung des Gehäuses 2 durch die Pumpe 9 entsprechend, wobei sowohl die absoluten Druckwerte als auch der zeitliche Verlauf variabel einstellbar sind. Durch die Messung der Ist-Werte mit Hilfe des zumindest einen Sensorabschnitts auf der Auslaßseite können die angestrebten Werte, mit denen sich tatsächlich einstellenden Größen verglichen und die Ansteuerung entsprechend verstellt werden.

Die Druckblase 1 bzw. der flexible Behälter und die daran angeformten Anschlußschläuche 3 und 4 mit den Hülsen 5 und 6 können aus dem Gehäuse 2 sowie den Sensorsegmenten 12 und 13 und den aktiven Klemmventilen 14 und 15 bei entsprechender Anordnung dieser Elemente ohne Schwierigkeiten entfernt werden. Das bedeutet, daß der mit Blut in Berührung kommende Teil der erfindungsgemäßen Vorrichtung schnell und problemlos ausgetauscht werden kann. Trotz aktiver Steuerung entfällt die Reinigung von Ventilelementen oder anderen nur mit großem Aufwand zu reinigen Bestandteilen. Das Gehäuse 2, die Sensorabschnitte 12 und 13 und die aktiven Klemmenwände 14 und 15 können dazu in entsprechender räumlicher Anordnung an einem Stativ befestigt werden und weisen dafür geeignete Anbringungseigenschaften auf.

## Patentansprüche

1. Vorrichtung zum Pumpen von Blut in einem extrakorporalen Blutkreislauf mit
a) einem flexiblen Behältnis (1), das
a1) einen Einlaßanschluß (3), an den eine Schlauchleitung des extrakorporalen Blutkreislaufs anschließbar oder gegebenenfalls angeschlossen ist und durch den das Blut in das flexible Behältnis zuführbar ist, und
a2) einen Auslaßanschluß (4), an den eine Schlauchleitung des extrakorporalen Blutkreislaufs anschließbar oder gegebenenfalls angeschlossen ist und durch den das Blut aus dem flexiblen Behältnis abführbar ist, aufweist,
b) einem druckdicht verschlossenen Gehäuse (2),
b1) in dem das flexible Behältnis (1) derart aufgenommen ist, daß die Anschlüsse (3,4) oder die daran angeschlossenen Schlauchleitungen durch in dem Gehäuse vorgesehene Öffnungen nach außen führen, und
b2) das einen Druckmittelanschluß (10) für die Zuführung eines den Innendruck des Gehäuses verändernden gasförmigen Druckmittels aufweist,
c) einer Pumpe (9), die an den Druckmittelanschluß (10) des Gehäuses (2) angeschlossen ist, für die Förderung des gasförmigen Druckmittels,
d) einem Sensorabschnitt (13),
d1) der an dem Auslaßanschluß (4) oder der daran angeschlossenen Schlauchleitung außerhalb des Gehäuses angeordnet ist und
d2) der Sensoren zur Erfassung von Druck und Durchflußmenge des Blutes aufweist,
e) zwei aktiven Klemmventilen (14, 15),
e1) die an den Anschlüssen (3, 4) des flexiblen Behälters (1) oder den daran angeschlossenen Schlauchleitungen außen angeordnet sind und
e2) die den Öffnungsquerschnitt der Anschlüsse bzw. der Schlauchleitungen durch mechanische Einwirkung von außen bestimmen, und
f) einer Steuereinheit (11), an die die Pumpe (9), die Sensoren des Sensorabschnitts (13) und die aktiven Klemmventile (14, 15) angeschlossen sind, zur Steuerung der Pumpe und der Klemmventile unter Berücksichtigung des von den Sensoren erfaßten Druckes und der von den Sensoren erfaßten Durchflußmenge, wobei die Steuereinheit die Schließ- und Öffnungszeiten und -geschwindigkeiten der Klemmventile steuert.

2. Vorrichtung nach Anspruch 1,
dadurch **gekennzeichnet**, daß
die aktiven Klemmventile (14, 15) ein bewegliches Verschlußelement (16, 17), das von einem elektrischen, elektromagnetischen, pneumatischen oder hydraulischen Antrieb (18, 19) bewegt wird, und ein Gegenlager (20, 21) aufweisen, das dem Verschlußelement (16, 17) in Bewegungsrichtung gegenüberliegend derart angeordnet ist, daß es mit dem Verschlußelement bei der Festlegung des Öffnungsquerschnitts der Anschlüsse oder der daran angeschlossenen Schlauchleitungen zusammenwirkt, und dessen Lage in Bezug auf die den kleinsten Öffnungsquerschnitt bestimmenden Stellung des Verschlußelements einstellbar ist.

3. Vorrichtung nach Anspruch 2,
dadurch gekennzeichnet, daß in den aktiven Klemmventilen (14, 15) eine Spindelschraube (22, 23) zur Festlegung der Lage des Gegenlagers (20, 21) vorgesehen ist.

4. Vorrichtung nach einem der vorangegangenen Ansprüche,
dadurch **gekennzeichnet**, daß
ein weiterer Sensorabschnitt (12), der an dem Einlaßanschluß (3) oder der daran angeschlossenen Schlauchleitung außen angeordnet ist, vorgesehen ist, der Sensoren zur Erfassung von Druck und Durchflußmenge des Blutes aufweist und dessen Sensoren an die Steuereinheit (11) angeschlossen sind.

5. Vorrichtung nach einem der vorangegangenen Ansprüche,
dadurch **gekennzeichnet**, daß
ein Blasendetektor zumindest in dem Sensorabschnitt (13) am Auslaßanschluß (4) oder der daran angeschlossenen Schlauchleitung vorgesehen und an die Steuereinheit (11) angeschlossen ist.

6. Vorrichtung nach einem der vorangegangenen Ansprüche,
dadurch **gekennzeichnet**, daß
auf der Auslaßseite des Gehäuses (2) das aktive Klemmventil (15) in Strömungsrichtung hinter dem Sensorabschnitt (13) angeordnet ist.

7. Vorrichtung nach einem der Ansprüche 4 bis 6,
dadurch **gekennzeichnet**, daß
auf der Einlaßseite des Gehäuses (2) das aktive Klemmventil (14) in Strömungsrichtung vor dem Sensorabschnitt (12) angeordnet ist.

8. Vorrichtung nach einem der vorangegangenen Ansprüche, dadurch gekennzeichnet, daß das Gehäuse (2) an den Öffnungen Dichtlemente (7, 8) aufweist.

9. Vorrichtung nach einem der vorangegangenen Ansprüche, dadurch gekennzeichnet, daß das Gehäuse (2) aus einem durchsichtigen Material hergestellt ist.

10. Vorrichtung nach einem der vorangegangenen Ansprüche, dadurch gekennzeichnet, daß das Gehäuse eine aufklappbare Kammer (24) ist, die einen Deckelteil (25) und einen Bodenteil (26) aufweist, die über ein Scharnier (29) miteinander schwenkbar verbunden sind und an ihren Kanten Dichtelemente aufweisen, und die mittels Kipphebelverschlüssen (30, 31) verschlossen wird.

11. Vorrichtung nach einem der vorangegangenen Ansprüche, dadurch gekennzeichnet, daß an den Anschlüssen (3, 4) oder den daran angschlossenen Schlauchleitungen im Bereich des Durchtritts durch die Öffnungen des Gehäuses (2) eine Hülse (5, 6), vorzugweise aus Hartplastik außen angeordnet und mit dem Auslaß oder der Schlauchleitung verbunden, vorzugweise verklebt ist.

12. Vorrichtung nach einem der vorangegangenen Ansprüche, dadurch gekennzeichnet, daß die Anschlüsse (3, 4) schlauchartig und einstückig mit dem flexiblen Behältnis (1) ausgebildet sind.

13. Vorrichtung nach einem der vorangegangenen Ansprüche, dadurch gekennzeichnet, daß das flexible Behältnis eine Druckblase (1) ist.

14. Vorrichtung nach Anspruch 13, dadurch gekennzeichnet, daß die Druckblase (1) und die Anschlüsse (3, 4) aus einem flexiblen Material, vorzugsweise Silikon hergestellt sind.

15. Vorrichtung nach einem der vorangegangenen Ansprüche, dadurch gekennzeichnet, daß der Steuereinheit (11) eine Ansteuerungssignal eines EKG-Gerätes zugeführt wird.

## Claims

1. Device for pumping blood in an extracorporeal blood circuit having
a) a flexible receptacle (1) which possesses
a1) an inlet connection (3) to which a hose line of the extracorporeal blood circuit can be connected or if need be is connected and through which the blood can be fed into the flexible receptacle, and
a2) an outlet connection (4) to which a hose line of the extracorporeal blood circuit can be connected or if need be is connected and through which the blood is carried away from the flexible receptacle,
b) a pressure-tight sealed housing (2)
b1) in which the flexible receptacle (1) is accommodated in such a way that the connections (3, 4) or the hose lines connected thereto lead to the outside through openings provided in the housing, and
b2) which possesses a pressure medium connection (10) for feeding a gaseous pressure medium altering the internal pressure of the housing,
c) a pump (9) which is connected to the pressure medium connection (10) of the housing (2) for the conveyance of the gaseous pressure medium,
d) a sensor section (13)
d1) which is arranged outside the housing on the outlet connection (4) or the hose line connected thereto and
d2) which possesses sensors for detecting the pressure and rate of flow of the blood,
e) two active clamping valves (14, 15)
e1) which are arranged outside on the connections (3, 4) of the flexible receptacle (1) or the hoses lines connected thereto and
e2) which determine the opening cross-section of the connections or of the hose lines by mechanical action from outside, and
f) a control unit (11) to which the pump (9), the sensors of the sensor section (13) and the active clamping valves (14, 15) are connected for controlling the pump and the clamping valves with respect to the pressure detected by the sensors and the flow rate detected by the sensors, the control unit controlling the closing and opening times and speeds of the clamping valves.

2. Device according to Claim 1,
characterised in that
the active clamping valves (14, 15) possess a movable closure member (16, 17) which is moved by an electric, electromagnetic, pneumatic or hydraulic drive (18, 19) and a counter-support (20, 21) which is arranged opposite the closure member (16, 17) in the direction of motion in such a way that it works together with the closure member in the determination of the opening cross-section of the connections or of the hose lines connected thereto and its position is adjustable with respect to the position of the closure member determining the smallest opening cross-section.

3. Device according to Claim 2,
characterised in that
in the active clamping valves (14, 15) a spindle screw (22, 23) for fixing the position of the counter-support (20, 21) is provided.

4. Device according to one of the preceding claims,
characterised in that
a further sensor section (12), which is arranged outside on the inlet connection (3) or the hose line connected thereto, is provided which possesses sensors for the detection of the pressure and rate of flow of the blood and the sensors of which are connected to the control unit (11).

5. Device according to one of the preceding claims,
characterised in that
a bubble detector is provided at least in the sensor section (13) on the outlet connection (4) or the hose line connected thereto and is connected to the control unit (11).

6. Device according to one of the preceding claims,
characterised in that
on the outlet side of the housing (2) the active clamping valve (15) is arranged behind the sensor section (13) in the direction of flow.

7. Device according to one of Claims 4 to 6,
characterised in that
on the inlet side of the housing (2) the active clamping valve (14) is arranged ahead of the sensor section (12) in the direction of flow.

8. Device according to one of the preceding claims
characterised in that
the housing (12) possesses sealing members (7, 8) at the openings.

9. Device according to one of the preceding claims,
characterised in that
the housing (2) is manufactured from a transparent material.

10. Device according to one of the preceding claims,
characterised in that
the housing is a chamber (24) capable of being swung open which possesses a cover part (25) and a base part (26) which are connected to one another in swivelling manner via a hinge (29) and possess sealing members at their edges and which are locked by means of rocker-arm locks (30, 31).

11. Device according to one of the preceding claims,
characterised in that
on the connections (3, 4) or the hose lines connected thereto in the region of the passage through the openings of the housing (2) a bush (5, 6) preferably made of rigid plastic is arranged outside and is connected, preferably bonded, to the outlet or the hose line.

12. Device according to one of the preceding claims,
characterised in that
the connections (3, 4) are constructed in the manner of hoses and in one piece with the flexible receptacle (1).

13. Device according to one of the preceding claims,
characterised in that
the flexible receptacle is a pressure vesicle (1).

14. Device according to Claim 13,
characterised in that
the pressure vesicle (1) and the connections (3, 4) are manufactured from a flexible material, preferably silicone.

15. Device according to one of the preceding claims,
characterised in that
a control signal from an ECG device is supplied to the control unit (11).

## Revendications

1. Dispositif de pompage de sang dans un circuit sanguin extracorporel, comportant
a) un récipient flexible (1), qui
a1) présente un raccordement d'entrée (3), auquel une conduite en tuyau appartenant au circuit sanguin extracorporel peut être raccordée ou, le cas échéant, est raccordée et au moyen duquel le sang peut être amené dans le récipient flexible, et
a2) un raccordement d'évacuation (4), auquel une conduite en tuyau appartenant au circuit sanguin extracorporel peut être raccordée ou, le cas échéant, est raccordée et au moyen duquel le sang, issu du récipient flexible, peut être évacué,
b) un boîtier (2) fermé de façon étanche à la pression,
b1) dans lequel le récipient flexible (1) est logé, de manière que les raccordements (3, 4) ou les conduites en tuyau y étant raccordées mènent vers l'extérieur, en passant par des ouvertures prévues dans le boîtier, et
b2) boîtier qui présente un raccordement de fluide sous pression (10) destiné à l'alimentation d'un fluide sous pression sous forme gazeuse, faisant changer la pression intérieure du boîtier,
c) une pompe (9), raccordée au raccordement de fluide sous pression (10) du boîtier (2) et destinée à véhiculer le fluide sous pression gazeux,
d) un tronçon à capteur (13)
d1) disposé sur le raccordement d'évacuation (4) ou sur la conduite en tuyau y étant raccordée, à l'extérieur du boîtier, et
d2) présentant des capteurs destinés à appréhender la pression et le débit de sang,
e) deux valves de serrage actives (14, 15),
e1) raccordées extérieurement aux raccordements (3, 4) du récipient flexible (1) ou des conduites en tuyau y étant raccordées, et
e2) déterminant l'aire de la section transversale d'ouverture des raccordements ou des conduites en tuyau, par action mécanique depuis l'extérieur, et
f) une unité de commande (11) à laquelle la pompe (9), les capteurs du tronçon à capteur (13) et les valves à serrage actives (14, 15) sont raccordés, pour assurer la commande de la pompe et des valves à serrage, en prenant en considération la pression appréhendée par les capteurs et le débit appréhendé par les capteurs, l'unité de commande modulant les temps et les vitesses de fermeture et d'ouverture des valves à serrage.

2. Dispositif selon la revendication 1,
caractérisé en ce que
les valves à serrage actives (14, 15) présentent un élément obturateur (16, 17) mobile, déplacé par un entraînement (18, 19) électrique, électromagnétique, pneumatique, ou hydraulique, et un contre-appui (20, 21) qui est disposé en regard de l'élément de fermeture (16, 17) dans la direction de déplacement, de manière à coopérer avec l'élément de fermeture lors de la détermination de la section transversale d'ouverture des raccordements ou des conduites en tuyau y étant raccordées, et dont la position est réglable par rapport à la position, déterminant l'aire de section transversale d'ouverture minimale, de l'élément de fermeture.

3. Dispositif selon la revendication 2, caractérisé en ce que dans les valves de serrage actives (14, 15) est prévue une vis de broche (22, 23) destinée à fixer la position du contre-appui (20, 21).

4. Dispositif selon l'une des revendications précédentes, caractérisé en ce qu'un autre tronçon à capteur (12), disposé extérieurement sur le raccordement d'entrée (3) ou la conduite en tuyau y étant raccordée, est prévu, qui présente des capteurs destinés à appréhender la pression et le débit du sang, et dont les capteurs sont raccordés à l'unité de commande (11);

5. Dispositif selon l'une des revendications précédentes, caractérisé en ce qu'un détecteur de bulles est prévu au moins dans le tronçon à capteur (13) au raccordement d'évacuation (4) ou sur la conduite en tuyau y étant raccordée, et est raccordé à l'unité de commande (11).

6. Dispositif selon l'une des revendications précédentes, caractérisé en ce que, du côté évacuation du boîtier (2), la valve à serrage active (15) est disposée, en observant dans la direction d'écoulement, en aval du tronçon à capteur (13).

7. Dispositif selon l'une des revendications 4 à 6, caractérisé en ce que du côté entrée du boîtier (2), la valve à serrage active (14) est disposée, en observant dans la direction d'écoulement, en amont du tronçon à capteur (12).

8. Dispositif selon l'une des revendications précédentes, caractérisé en ce que le boîtier 2) présente des éléments d'étanchéité (7, 8) sur les ouvertures.

9. Dispositif selon l'une des revendications précédentes, caractérisé en ce que le boîtier (2) est fabriqué en un matériau transparent.

10. Dispositif selon l'une des revendications précédentes, caractérisé en ce que le boîtier est une chambre (24) ouvrable par rabattement, qui présente une partie formant couvercle (25) et une partie formant fond (26), reliées de façon pivotante ensemble par une charnière (29) et présentant sur leurs arêtes des éléments d'étanchéité et étant obturées au moyen de fermetures à levier basculant (30, 31).

11. Dispositif selon l'une des revendications précédentes, caractérisé en ce qu'aux raccordements (3, 4) ou aux conduites en tuyau y étant raccordées, dans la zone du passage à travers les ouvertures du boitier (2), une douille (5, 6), de préférence en matière plastique dure, est disposée extérieurement et reliée à l'évacuation ou à la conduite formant tuyau, de préférence par collage.

12. Dispositif selon l'une des revendications précédentes, caractérisé en ce que les raccordements (3, 4) sont réalisés à la façon d'un tuyau et d'une seule pièce avec le récipient flexible (1).

13. Dispositif selon l'une des revendications précédentes, caractérisé en ce que le récipient flexible est une vessie à pression (1).

14. Dispositif selon la revendication 13, caractérisé en ce que la vessie à pression (1) et les raccordements (3, 4) sont fabriqués en un matériau flexible, de préférence en silicone.

15. Dispositif selon l'une des revendications précédentes, caractérisé en ce qu'à l'unité de commande (11) est amené un signal d'attaque venant d'un appareil d'électro-cardiogramme (ECG).
